# EUROPÄISCHE PATENTANMELDUNG

(11) **EP 1 844 796 A2**
(43) Veröffentlichungstag der Anmeldung: **17.10.2007**
(21) Anmeldenummer: 07105905.9
(22) Anmeldetag: 10.04.2007
(51) Int. Cl.: A61L 9/22, B01D 53/86, F24F 3/16, G03G 21/20, F24F 13/28

(54) **Verfahren und Vorrichtung zur Aufbereitung der Abluft eines elektrischen oder elektronischen Geräts, insbesondere eines Kopierers oder eines Druckers**

(30) Priorität: 13.04.2006 DE 102006017474
(71) Anmelder: Gesellschaft für sicherheits- und brandschutz-, 90411 Nürnberg (DE)
(72) Erfinder: Laskowski, Rainer, 32130, Enger (DE); Kolb, Thomas, 90408, Nürnberg (DE)
(74) Vertreter: Zech, Stefan Markus

(57) **Zusammenfassung**

Die Erfindung betrifft ein Verfahren zur Aufbereitung der Abluft eines elektrischen oder elektronischen Geräts (1), insbesondere eines Kopierers oder eines Druckers, bei dem die Abluft entlang eines Strömungsweges (2) geleitet und aus dem elektrischen oder elektronischen Gerät (1) ausgeleitet wird, wobei die Abluft einer Reinigung und/oder Aufbereitung unterzogen wird. Um die Umweltbelastung durch die Abluft zu reduzieren, ist erfindungsgemäß vorgesehen, dass die Abluft durch mindestens zwei unterschiedliche Reinigungs- und/oder Aufbereitungssysteme (3, 4, 5) geleitet wird, die aus der Gruppe eines Filterelements (3), eines Elements (4) mit katalytischer Wirkung und eines Ionisators (5) ausgewählt sind. Des weiteren betrifft die Erfindung eine Vorrichtung zur Aufbereitung der Abluft eines elektrischen oder elektronischen Geräts, insbesondere eines Kopierers oder eines Druckers.

## Beschreibung

Die Erfindung betrifft ein Verfahren zur Aufbereitung der Abluft eines elektrischen oder elektronischen Geräts, insbesondere eines Kopierers oder eines Druckers, bei dem die Abluft entlang eines Strömungsweges geleitet und aus dem elektrischen oder elektronischen Gerät ausgeleitet wird, wobei die Abluft einer Reinigung und/oder Aufbereitung unterzogen wird. Des weiteren betrifft die Erfindung eine Vorrichtung zur Aufbereitung der Abluft eines elektrischen oder elektronischen Geräts, insbesondere eines Kopierers oder eines Druckers.

Ein Kopierer und ein Drucker erzeugen im Betrieb diverse Schadstoffe, die umweltbelastend sind. Dabei sind insbesondere Ozon, Benzol und Styrol zu nennen, deren Menge bei der Freigabe des Geräts ermittelt wird; die Freigabe kann nur erteilt werden, wenn gewisse Höchstmengenströme nicht überschritten werden. Ferner erzeugt der Kopierer bzw. der Drucker im Betrieb Feinstaub, der ebenfalls umweltbelastend ist.

Aus der DE 20 2004 012 352 U1 ist eine Anordnung bekannt, bei der die Abluft vor dem Verlassen des elektrischen Geräts dadurch aufbereitet wird, dass es vor dem Ausströmen durch einen Aktivkohlefilter geleitet und anschließend mit Ionen angereichert wird. Damit soll erreicht werden, reine Luft zu ionisieren und dabei, um eine naturgleiche Ionenzahl in Räumen mittlerer Größe aufbauen zu können, eine entsprechende Ionenzahl zu erzeugen, weil Ionen ihre Ladung an Oberflächen aller Art und an luftgetragene Kleinstpartikel, Bakterien, Viren und Nanostäube abgeben. Daher sieht die dort beschriebene Lösung vor, dass die Luft in einem mehrstufigen Verfahren aufbereitet wird. Die Abluft wird dabei zunächst in einem mechanischen Filter vorgereinigt. Dann erfolgt eine Anreicherung mit Ozon und Ionen. Weiterhin wird die Abluft dann durch ein Aktivkohlefilter geleitet, wodurch sie von Bakterien, Viren, Proteinen und organischen Substanzen bzw. Dämpfen gereinigt werden soll. Schließlich wird die so vorbehandelte Abluft mit überwiegend negativen Ionen angereichert. Hierzu ist vorgesehen, dass durch Entladungsvorgänge und durch Elektronenaustritt aus einer Elektrode die vorbeiströmende Abluft ionisiert wird und dass negative Ionen erzeugt werden, die durch ein Schutzgitter aus dem Gerät austreten.

Die DE 10 2004 003 269 A1 beschreibt ein Verfahren und eine Vorrichtung zur Reduzierung von Keimen und VOC-Lasten (Volatile Organic Compounds) in der Raumluft, auf Flächen im Raum und den an den Raum angeschlossenen Belüftungsanlagen sowie zur Aufrechterhaltung eines naturadäquaten Verhältnisses von Sauerstoffionen und Ozon im Raum über die Luftzuführung oder zentrale Lüftungsanlagen oder über Luftumwälzung durch separate, im Raum platzierte, kombinierte Ionen- und Ozonerzeugungsgeräte. Dabei wird in einem Luftleitkanal ein Aktivkohlefilter und ein Ozonisierungs- und Ionisationsmodul in Kombination mit einem Leistungsteil und einer Steuereinheit angeordnet. Dabei kann vorgesehen werden, dass zwischen den Leistungsteilen ein Sorptionskatalysator vorgesehen wird, in dem die VOC- und Keimlasten zwischengespeichert und mittels des Ozons und die daraufhin erfolgende Bildung von OH-Radikalen und Wasserstoffsuperoxidation aus Wasser, das über die relative Luftfeuchtigkeit gebunden wird, im Sorptionskatalysator aboxidiert werden. Der Sorptionskatalysator ist dabei als Aktivkohlefilter ausgeführt.

Aus der DE 199 02 825 A1 ist ein Reaktor zur Umwandlung von Ozon in Sauerstoff bekannt, wobei die Umwandlung des Ozons in Sauerstoff katalytisch erfolgt. Dabei kommen Katalysatoren unter Verwendung von Mangandioxid und Bleidioxyd zum Einsatz.

Ähnliche Lösungen sind aus der DE 28 50 154 A1, aus der DE 26 53 789 A1 und aus der DE 24 35 820 A1 bekannt.

Es hat sich gezeigt, dass trotz der vorbekannten Verfahren und Vorrichtungen insbesondere der Ausstoß an PM10 Feinstaub, d. h. an Feinstaub mit Partikelgrößen kleiner als 10 µm, Benzol und anderen Schadstoffen immer noch relativ hoch ist, was eine entsprechende Gesundheitsgefährdung nach sich ziehen kann.

Der Erfindung liegt daher die **Aufgabe** zugrunde, ein Verfahren zur Aufbereitung der Abluft eines elektrischen oder elektronischen Geräts, insbesondere eines Kopierers oder eines Druckers, der eingangs genannten Art sowie eine entsprechende Vorrichtung so weiterzuentwickeln, dass die Umweltbelastung weiter reduziert werden kann. Der Ausstoß an Schadstoffen soll also weiter eingedämmt werden.

Die **Lösung** dieser Aufgabe durch die Erfindung ist verfahrensgemäß dadurch gekennzeichnet, dass die Abluft des elektrischen bzw. elektronischen Geräts, insbesondere des Kopierers oder Druckers, durch mindestens zwei unterschiedliche Reinigungs- und/oder Aufbereitungssysteme geleitet wird, die aus der Gruppe eines Filterelements, eines Elements mit katalytischer Wirkung und eines Ionisators ausgewählt sind.

Eine Ausgestaltung der Erfindung sieht dabei vor, dass die Abluft in dieser Reihenfolge durch ein Filterelement und durch ein Element mit katalytischer Wirkung oder durch einen Ionisator geleitet wird.

Eine andere Lösung stellt darauf ab, dass die Abluft in dieser Reihenfolge durch ein Element mit katalytischer Wirkung und durch einen Ionisator geleitet wird.

Besonders bevorzugt ist jedoch vorgesehen, dass die Abluft in dieser Reihenfolge durch ein Filterelement, ein Element mit katalytischer Wirkung und durch einen Ionisator geleitet wird.

Die Filterung der Abluft erfolgt dabei bevorzugt durch einen Aktivkohlefilter.

Es hat sich herausgestellt, dass ein Aktivkohlefilter selber gegebenenfalls Feinstaub erzeugen kann, so dass sich die Nachschaltung eines Katalysators und eines Ionisators als besonders vorteilhaft erweist. Dies gilt namentlich dann, wenn der Katalysator eine hohe Speicherwirkung für Ozon aufweist.

Die Vorrichtung zur Aufbereitung der Abluft hat einen Strömungsweg, entlang dem die Abluft geleitet und aus dem Gerät ausgeleitet wird. Die Vorrichtung zeichnet sich erfindungsgemäß dadurch aus, dass im Strömungsweg der Abluft mindestens zwei unterschiedliche Reinigungs- und/oder Aufbereitungssysteme angeordnet sind, die aus der Gruppe eines Filterelements, eines Elements mit katalytischer Wirkung und eines Ionisators ausgewählt sind.

Analog zum erfindungsgemäßen Verfahren ist gemäß einer Ausgestaltung vorgesehen, dass im Strömungsweg in dieser Reihenfolge ein Filterelement und ein Element mit katalytischer Wirkung oder ein Ionisator angeordnet sind.

Alternativ kann vorgesehen sein, dass im Strömungsweg in dieser Reihenfolge ein Element mit katalytischer Wirkung und ein Ionisator angeordnet sind.

Besonders bevorzugt sind alternativ im Strömungsweg in dieser Reihenfolge ein Filterelement, ein Element mit katalytischer Wirkung und ein Ionisator angeordnet.

Das Element mit katalytischer Wirkung ist vorzugsweise ein Mangandioxid-Katalysator. Als katalytisches Element vorgesehen werden kann aber auch ein Katalysator, der einen Aluminiumoxidträger aufweist, auf dem Mangandioxid und Kupferoxid aufgebracht ist.

Die Effizienz des Katalysators wird erhöht, wenn eine Wirkoberfläche in Wabenstruktur vorgesehen wird.

Vorzugsweise ist ein Ventilator vorgesehen, der die Abluft durch den Strömungsweg fördert. Der Ventilator kann dabei in Strömungsrichtung vor den Reinigungs- und/oder Aufbereitungssystemen angeordnet werden.

Die Erfindung besteht zusammengefasst also darin, dass gemäß einer bevorzugten Ausgestaltung stromab des Aktivkohlefilters ein Katalysator und daran anschließend ein Ionisator angeordnet wird. Alternativ kann auch vorgesehen sein, dass eine Kombination von Katalysator und Ionisator unabhängig von einem vorgeschalteten Aktivkohlefilter zum Einsatz kommt. Weiter alternativ kann auch ein Aktivkohlefilter vorgesehen werden, der in Kombination mit einem Katalysator oder einem Ionisator betrieben wird.

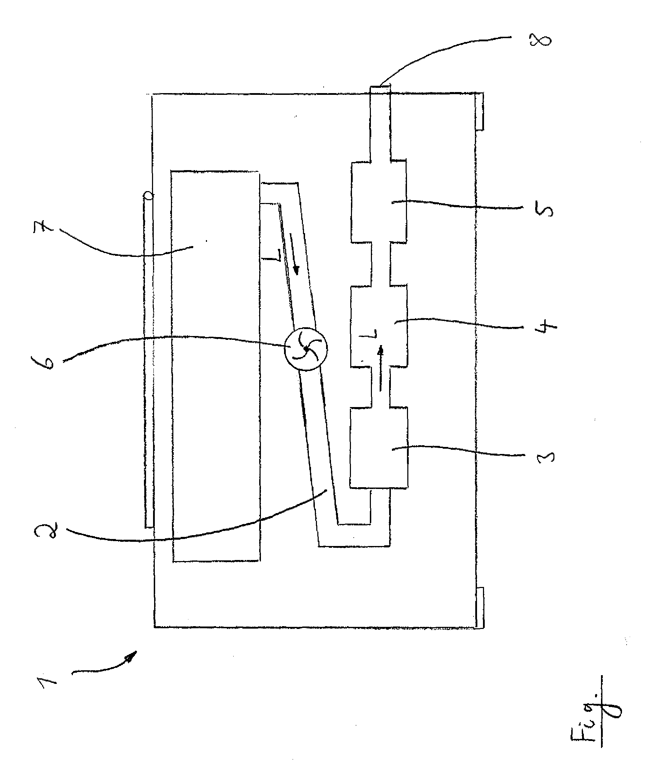

In der Zeichnung ist ein Ausführungsbeispiel der Erfindung dargestellt. Die einzige Figur zeigt schematisch den Schnitt durch einen Kopierer, der mit einer Ausführungsform der erfindungsgemäßen Vorrichtung zur Aufbereitung der Abluft ausgestattet ist.

Die Figur zeigt einen Kopierer 1, der eine Kopiereinheit 7 aufweist, die nicht näher dargestellt und die in bekannter Weise aufgebaut ist. Die Kopiereinheit 7 erzeugt während des Betriebs Abluft L, die über einen Strömungsweg 2 aus dem Kopierer 1 über einen Luftauslass 8 abgeleitet werden muss. Hierfür ist ein Ventilator 6 vorgesehen, der an geeigneter Stelle in den Strömungsweg 2 integriert ist.

Die Abluft L ist in Strömungsrichtung hinter der Kopiereinheit 7 mit umweltbelastenden bzw. gesundheitsbelastenden Partikeln versehen, wobei insbesondere Feinstaub und Benzol besonders zu erwähnen sind.

Um diese Partikel zu eliminieren, ist im Strömungsweg 2 ein mehrstufiges Reinigungs- und/oder Aufbereitungssystem 3, 4, 5 vorgesehen. Dieses System weist zunächst einen Aktivkohlefilter 3 auf, der an sich bekannt und gebräuchlich ist. An diesen schließt sich in Strömungsrichtung der Abluft L ein Katalysator 4 an. Hieran wiederum schließt sich ein Ionisator 5 an.

Für den Katalysator 4 hat sich ein Mangandioxidelement bzw. Mangandioxid- und Kupferoxid auf einem Aluminiumoxidträger besonders bewährt. Eine Ausbildung der katalytischen Oberfläche als Wabenstruktur schafft eine hohe Effizienz des Katalysators.

Insbesondere durch die Kombination der drei Elemente 3, 4 und 5 des Reinigungs- und/oder Aufbereitungssystem wird erreicht, dass die Abluft L am Luftauslass 8 des Kopierers eine sehr viel geringere Umweltbelastung bzw. Gesundheitsbelastung aufweist, als ohne die Kombination der vorgeschlagenen Elemente.

Besonders vorteilhaft ist die vorgeschlagene Ausgestaltung, weil der Aktivkohlefilter 3 mitunter selber nicht unerhebliche Mengen an Feinstaub erzeugt, die durch die weiteren Elemente 4, 5 eliminiert werden können, d. h. die Nachschaltung des Katalysators 4 und des Ionisators 5 ist sehr zweckmäßig.

### Bezugszeichenliste

- 1: Kopierer, Drucker
- 2: Strömungsweg
- 3, 4, 5: Reinigungs- und/oder Aufbereitungssysteme
- 3: Filterelement
- 4: Katalysator
- 5: Ionisator
- 6: Ventilator
- 7: Kopiereinheit
- 8: Luftauslass

- L: Abluft

## Patentansprüche

1. Verfahren zur Aufbereitung der Abluft eines elektrischen oder elektronischen Geräts (1), insbesondere eines Kopierers oder eines Druckers, bei dem die Abluft entlang eines Strömungsweges (2) geleitet und aus dem elektrischen oder elektronischen Gerät (1) ausgeleitet wird, wobei die Abluft einer Reinigung und/oder Aufbereitung unterzogen wird,
**dadurch gekennzeichnet,**
**dass** die Abluft durch mindestens zwei unterschiedliche Reinigungs- und/oder Aufbereitungssysteme (3, 4, 5) geleitet wird, die aus der Gruppe eines Filterelements (3), eines Elements (4) mit katalytischer Wirkung und eines Ionisators (5) ausgewählt sind.

2. Verfahren nach Anspruch 1, **dadurch gekennzeichnet, dass** die Abluft in dieser Reihenfolge durch ein Filterelement (3) und durch ein Element (4) mit katalytischer Wirkung oder durch einen Ionisator (5) geleitet wird.

3. Verfahren nach Anspruch 1, **dadurch gekennzeichnet, dass** die Abluft in dieser Reihenfolge durch ein Element (4) mit katalytischer Wirkung und durch einen Ionisator (5) geleitet wird.

4. Verfahren nach Anspruch 1, **dadurch gekennzeichnet, dass** die Abluft in dieser Reihenfolge durch ein Filterelement (3), ein Element (4) mit katalytischer Wirkung und durch einen Ionisator (5) geleitet wird.

5. Verfahren nach einem der Ansprüche 1 bis 4, **dadurch gekennzeichnet, dass** die Filterung der Abluft durch einen Aktivkohlefilter (3) erfolgt.

6. Vorrichtung zur Aufbereitung der Abluft eines elektrischen oder elektronischen Geräts (1), insbesondere eines Kopierers oder eines Druckers, mit einem Strömungsweg (2), entlang dem die Abluft geleitet und aus dem elektrischen oder elektronischen Gerät (1) ausgeleitet wird, insbesondere zur Durchführung des Verfahrens nach einem der Ansprüche 1 bis 5,
**dadurch gekennzeichnet,**
**dass** im Strömungsweg (2) der Abluft mindestens zwei unterschiedliche Reinigungs- und/oder Aufbereitungssysteme (3, 4, 5) angeordnet sind, die aus der Gruppe eines Filterelements (3), eines Elements (4) mit katalytischer Wirkung und eines Ionisators (5) ausgewählt sind.

7. Vorrichtung nach Anspruch 6, **dadurch gekennzeichnet, dass** im Strömungsweg (2) in dieser Reihenfolge ein Filterelement (3) und ein Element (4) mit katalytischer Wirkung oder ein Ionisator (5) angeordnet sind.

8. Vorrichtung nach Anspruch 6, **dadurch gekennzeichnet, dass** im Strömungsweg (2) in dieser Reihenfolge ein Element (4) mit katalytischer Wirkung und ein Ionisator (5) angeordnet sind.

9. Vorrichtung nach Anspruch 6, **dadurch gekennzeichnet, dass** im Strömungsweg (2) in dieser Reihenfolge ein Filterelement (3), ein Element (4) mit katalytischer Wirkung und ein Ionisator (5) angeordnet sind.

10. Vorrichtung nach einem der Ansprüche 6 bis 9, **dadurch gekennzeichnet, dass** das Filterelement (3) ein Aktivkohlefilter ist.

11. Vorrichtung nach einem der Ansprüche 6 bis 10, **dadurch gekennzeichnet, dass** das Element (4) mit katalytischer Wirkung ein Mangandioxid-Katalysator ist.

12. Vorrichtung nach einem der Ansprüche 6 bis 10, **dadurch gekennzeichnet, dass** das Element (4) mit katalytischer Wirkung ein Katalysator ist, der einen Aluminiumoxidträger aufweist, auf dem Mangandioxid und/oder Kupferoxid aufgebracht ist.

13. Vorrichtung nach einem der Ansprüche 6 bis 12, **dadurch gekennzeichnet, dass** das Element (4) mit katalytischer Wirkung eine Wirkoberfläche in Wabenstruktur aufweist.

14. Vorrichtung nach einem der Ansprüche 6 bis 13, **dadurch gekennzeichnet, dass** ein Ventilator (6) vorgesehen ist, der die Abluft durch den Strömungsweg (2) fördert.

15. Vorrichtung nach Anspruch 14, **dadurch gekennzeichnet, dass** der Ventilator (5) in Strömungsrichtung vor den Reinigungs- und/oder Aufbereitungssystemen (3, 4, 5) angeordnet ist.
